# EUROPEAN PATENT APPLICATION

(11) **EP 4 327 780 A1**
(43) Date of publication of application: **28.02.2024**
(21) Application number: 22790963.7
(22) Date of filing: 15.04.2022
(51) Int. Cl.: A61F 2/24

(54) **MANIPULATABLE VALVE CLIP SYSTEM**

(30) Priority: 21.04.2021 CN 202110427275
(71) Applicant: Shanghai Shenqi Medical Technology Co., Ltd., Shanghai 201201 (CN)
(72) Inventor: WANG, Sen, Shanghai 201201 (CN); SONG, Rui, Shanghai 201201 (CN); WANG, Zhichen, Shanghai 201201 (CN); DAI, Zhihao, Shanghai 201201 (CN); ZHOU, Guolei, Shanghai 201201 (CN); HUANG, Zhongrong, Shanghai 201201 (CN)
(74) Representative: Cabinet Laurent & Charras
(86) International application number: PCT/CN2022/087045
(87) International publication number: WO 2022/222855

(57) **Abstract**

A manipulatable valve clip system, comprising a delivery sheath catheter (10), a rod (20), a control wire (30) and a mitral valve repair device (40). The mitral valve repair device (40) comprises a central base (41) and proximal clips (42). The rod (20) is connected to the delivery sheath catheter (10), the central base (41) is arranged on the rod (20), and the proximal clips (42) are mounted on the central base (41). The delivery sheath catheter (10) has a first through hole (11) and a second through hole (12) which are symmetrically arranged, and the rod (20) is provided with a third through hole (21). The control wire (30) passes through the first through hole (11) to connect to the proximal clip (42) on one side of the central base (41), the control wire (30) also passes through the third through hole (21) to connect to the proximal clip (42) on the other side of the central base (41), the control wire (30) also passes through and out of the delivery sheath catheter (10) via the second through hole (12), and the control wire (30) is configured to be able to interlock with the rod (20). The manipulatable valve clip system can improve the safety of an interventional surgical operation for mitral regurgitation.

## Description

### TECHNICAL FIELD

The present application relates to the technical field of medical devices, and particularly to a manipulatable valve clamping system.

### BACKGROUND

Mitral regurgitation is the most common heart valve disease, and the mitral regurgitation is characterized by retrograde flow from the left ventricle to the left atrium of the heart through the dysfunctional mitral valve. During the normal systolic (systole) cycle, the mitral valve acts as a check valve to prevent the return of oxygenated blood into the left atrium. Thus, oxygenated blood is pumped through the aortic valve to the aorta. Valve regurgitation significantly reduces the pumping efficiency of the heart, thereby placing the patient at risk of severe, progressive heart failure.

The most common treatment for mitral regurgitation relies on valve replacement or repair, including valve leaflet and ring remodeling. The latter is commonly referred to as valvuloplasty. The latest technique for mitral valve repair relies on the suturing of adjacent segments of the valve leaflets opposite to each other together, which is known as the "bow-tie" or "edge-to-edge" technique. Although all of these techniques can be very effective, they usually rely on open-heart surgery. The patient's chest is usually to be opened through a sternotomy, and the patient is placed on cardiopulmonary bypass. The need to simultaneously open the chest and place the patient on cardiopulmonary bypass is traumatic and has associated high incidence rate and mortality rate.

In recent years, with the breakthrough of valve interventional therapy technology, mitral regurgitation interventional therapy devices have become one of the key research and development directions of cardiovascular instruments at home and abroad. The traditional valve clamping system is mostly double-arm grasping without considering the situation that only one valve leaflet is grasped during an operation, causing only two valve leaflets being released at the same time for re-grasping when the operation encounters this situation, which is difficult to clamp and prone to damage the valve leaflet. Therefore, it needs to design a valve clamping system that can operate single-side of the valve leaflet separately. However, the conventional valve clamping system that can operate valve leaflet separately uses two control wires to control two proximal clips respectively. During multiple grasping, the two control wires are easily tangled together, which will increase the operation difficulty, affect the operation time and increase the operation risk.

### SUMMARY

In view of this, the present application discloses a manipulatable valve clamping system, which is possible to improve the safety of mitral regurgitation intervention.

A manipulatable valve clamping system includes: a delivery sheath, a rod, a control wire, and a mitral valve repair device. The mitral valve repair device comprises a central base and proximal clips. The rod is connected to the delivery sheath. The central base is arranged on the rod. The proximal clips are mounted on the central base. The delivery sheath is provided with a first through hole and a second through hole arranged symmetrically. The rod is provided with a third through hole. The control wire passes through the first through hole and is connected to the proximal clip on a side of the central base. The control wire further passes through the third through hole and is connected with the proximal clip on the other side of the central base. The control wire further passes out of the delivery sheath through the second through hole, and the control wire is configured to be interlocked with the rod.

In an embodiment, the mitral valve repair device further includes a distal clip and an adjusting mechanism. The adjusting mechanism includes a transmission rod and an adjusting arm. The transmission rod extends through the rod and the central base respectively and is connected to the adjusting arm. One end of the distal clip is connected to the central base, and the other end of the distal clip is connected to the adjusting arm.

In an embodiment, an aperture of the third through hole is equal to or slightly larger than a wire diameter of the control wire, and the control wire located in the rod is wrapped around a portion of the transmission rod.

In an embodiment, the transmission rod is provided with a keyway, and the control wire located in the rod passes through the keyway.

In an embodiment, the manipulatable valve clamping system further includes a fixing sleeve. The fixing sleeve is sleeved on an outer side of the rod. The fixing sleeve is provided with an opening for the control wire to pass through. When the control wire is interlocked with the rod, the opening is staggered with the third through hole.

In an embodiment, the fixing sleeve is provided with a first rubber washer at the opening.

In an embodiment, the rod is provided with external threads, the fixing sleeve is provided with internal threads, and the fixing sleeve is thread-connected with the rod.

In an embodiment, the rod is provided with a second rubber washer at the third through hole.

In an embodiment, the proximal clip is provided with a plurality of connection holes, and the control wire sequentially passes through adjacent two connection holes to connect with the proximal clip.

In an embodiment, the mitral valve repair device further includes a binding wire that ties the control wire to the proximal clip.

In an embodiment, the rod is provided with the plurality of third through holes, and the plurality of third through holes are spaced apart along an axial direction of the rod.

It can be seen from the above technical solution that the present application discloses a manipulable valve clamping system. The above-mentioned manipulable valve clamping system uses only one control wire to control two proximal clips of the mitral valve repair device, and the control wire is configured to be interlockable with the rod. When the control wire and the rod are interlocked, operating the control wire on one side of the rod separately does not affect the control wire on the other side of the rod. Not only both ends of the control wire can be operated to operate the two proximal clips simultaneously, but also one end of the control wire can be operated separately to operate the one proximal clip separately. Therefore, the valve leaflet can be simultaneously grasped on both sides and separately grasped on one side by one control wire, which improves the grasping capability of the proximal clip. Since a single control wire is used, one control wire is respectively connected to two proximal clips respectively, the control wire is placed on both sides of the rod respectively, and pass through the two lumens of the delivery sheath. During an operation, tightening and pushing is safer, and the control wires are not become entangled and overlapped with each other. Meanwhile, fewer control wires are used, which also reduces the possibility of the control wires 30 getting entangled with the chordae, thereby improving operation safety. Moreover, since the rod is harder than the control wire, the force of the control wire will not deform the rod when the valve leaflet is grasped on one side, and the bending of the delivery sheath and the swinging of the manipulable valve clamping system can be avoided, which is conducive to simplifying the complexity of the operation, saving the operation time, and further ensuring the operation safety.

### BRIEF DESCRIPTION OF THE DRAWINGS

In order to more clearly explain the technical solutions in embodiments of the present application or in the conventional art, the following will briefly illustrate the drawings required in the description of the embodiments or the conventional art. Obviously, the drawings in the following description are only embodiments of the present application. For those of ordinary skill in the art, other drawings can be obtained according to the disclosed drawings, without any creative efforts.
FIG. 1 is a schematic view of a manipulable valve clamping system in an embodiment.
FIG. 2 is a partial cross-sectional view of a manipulable valve clamping system in an embodiment.
FIG. 3 is a schematic view of a proximal clip in an embodiment.
FIG. 4 is a schematic view of a delivery sheath in an embodiment.
FIG. 5 is a schematic view of a rod in an embodiment.
FIG. 6 is a schematic view of a wire and a rod in a locking state in an embodiment.
FIG. 7 is a schematic view of an application of a manipulable valve clamping system in an embodiment.
FIG. 8 is a schematic view of a rod in another embodiment.
FIG. 9 is a schematic view of a wire and a rod in a locking state in another embodiment.
FIG. 10 is a schematic view of a wire and a rod in a locking state in yet another embodiment.

Description of the Reference Numerals:
10-delivery sheath, 11-first through hole, 12-second through hole, 13-main lumen, 20-rod, 21-third through hole, 30-control wire, 40-mitral valve repair device, 41-cetral base ,42-proximal clip, 421-collection hole, 43-distal clip, 44-transmission rod, 441-keyway, 45-adjusting arm, 50-second rubber washer, 60-fixing sleeve, 61-opening.

### DETAILED DESCRIPTION OF THE EMBODIMENTS

The solutions of the present application will be described below in conjunction with the drawings and specific embodiments.

In the description of the present application, it is to be understood that orientation or position relationships that are indicated by the terms "on", "under", "front", "rear", "left", "right", "vertical", "horizontal", "top", "bottom", "inside", "outside", and the like are orientation or position relationships shown based on the accompany drawings, and are merely for convenience of the description of the present application and simplifying description, rather than indicating or implying that the indicated appliance or element must have a particular orientation or being constructed and operated in a particular orientation, and are therefore not to be construed as limitation of the present application.

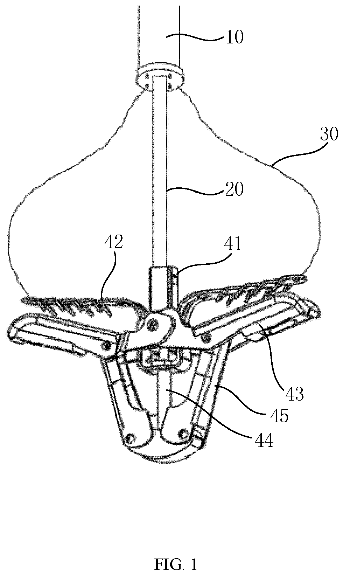

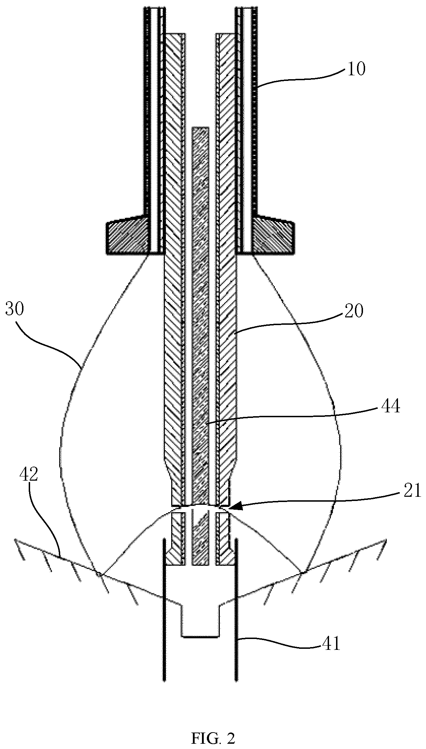

Referring to FIG. 1 to FIG. 7 at the same time, a manipulable valve clamping system of an embodiment includes a delivery sheath 10, a rod 20, a control wire 30, and a mitral valve repair device 40. The mitral valve repair device 40 includes a central base 41 and proximal clips 42. The rod 20 is connected to the delivery sheath 10. The central base 41 is arranged on the rod 20. The proximal clips 42 are mounted on the central base 41. The delivery sheath 10 is provided with a first through hole 11 and a second through hole 12 which are arranged symmetrically. The rod 20 is provided with a third through hole 21. The control wire 30 passes through the first through hole 11 and is connected to the proximal clip 42 on a side of the central base 41. The control wire 30 further passes through the third through hole 21 and is connected to the proximal clip 42 on the other side of the central base 41. The control wire 30 further passes out of the delivery sheath 10 through the second through hole 12. The control wire 30 is configured to be interlockable with the rod 20.

Specifically, the proximal clip 42 can be opened or closed with respect to the central base 41, and the control wire 30 controls the proximal clip 42 to be opened or closed with respect to the central base 41. In a normal state, the proximal clip 42 is expanded outwardly with respect to the central base 41, and in a locked state, the proximal clip 42 is tightened by the control wire 30 to be tightly attached to the central base 41. Further, the lower surface of the proximal clip 42 is provided with barbs, which facilitates the clamping of the valve leaflets by the proximal clip 42. In addition, the rod 20 is made of nickel-titanium rod or other memory alloy materials, which can be selected and adjusted according to actual needs.

Further, the control wire 30 is a single silk, and the hardness of the control wire 30 is less than the hardness of the rod 20. Specifically, the control wire 30 may be, but is not limited to, selected from a nickel-titanium wire, a stainless-steel wire, or a high-strength polymer wire. In the case that the control wire 30 is a polymer wire, it may be a single wire twisted with multiple strands. For convenience of description, the following embodiments takes the control wire 30 as the nickel-titanium wire for description.

The above-mentioned manipulable valve clamping system uses only one control wire 30 to control the two proximal clips 42 of the mitral valve repair device 40, and the control wire 30 is configured to be interlockable with the rod 20. When the control wire 30 and the rod 20 are interlocked, operating the control wire 30 on one side of the rod 20 separately does not affect the control wire 30 on the other side of the rod 20. Not only both ends of the control wire 30 can be operated at the same time to operate the two proximal clips 42, but also one end of the control wire 30 can be operated separately to operate one proximal clip 42 separately. Therefore, the valve leaflet can be simultaneously grasped on both sides and separately grasped on one side by one control wire 30, which improves the grasping capability of the proximal clips 42. Since a single control wire 30 is used, one control wire 30 is connected to two proximal clips 42 respectively, the control wire 30 is placed on both sides of the rod 20 respectively, and pass through two lumens of the delivery sheath 10. During the operation, tightening and pushing is safer, and the control wires 30 are not become entangled and overlapped with each other. Meanwhile, fewer control wires 30 are used, which also reduces the possibility of the control wires 30 getting entangled with the chordae, thereby improving operation safety. Moreover, since the rod 20 is harder than the control wire 30, the force of the control wire 30 will not deform the rod 20 when the valve leaflet is grasped on one side, and the bending of the delivery sheath 10 and the swinging of the manipulable valve clamping system can be avoided, which is conducive to simplifying the complexity of the operation, saving the operation time, and further ensuring the operation safety.

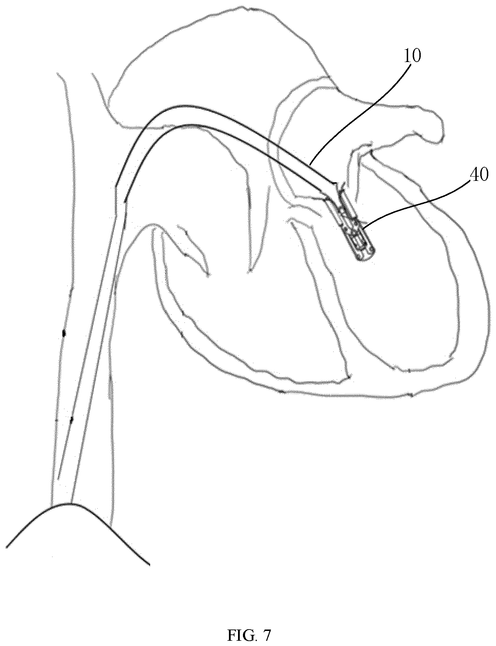

In an embodiment, the mitral valve repair device 40 further includes a distal clip 43 and an adjusting mechanism. The adjusting mechanism includes a transmission rod 44 and an adjusting arm 45. The transmission rod 44 extends through the rod 20 and the central base 41 respectively and is connected to the adjusting arm 45. One end of the distal clip 43 is connected to the central base 41, and the other end of the distal clip 43 is connected to the adjusting arm 45. Specifically, the action of the transmission rod 44 drives the distal clip 43 to open or close relative to the central base 41 through the adjusting arm 45. In the initial state, both of the proximal clip 42 and the distal clip 43 are closed relative to the central base 41, and the mitral valve repair device 40 is placed at a position (as shown in FIG. 7) where the mitral valve cannot be properly closed. The transmission rod 44 is operated to slide axially with respect to the central base 41. Under the pulling of the transmission rod 44, the adjusting arm 45 drives the distal clip 43 to expand relative to the central base 41. When the distal clip 43 is expanded at a lesion position below the valve, the control of the control wire 30 on the proximal clip 42 is released, and the proximal clip 42 is released to expand to be close to the distal clip 43. The proximal clip 42 and the distal clip 43 are close to each other to clamp the valve leaflet therebetween. When the valve leaflet on one side is not firmly clamped, the control wire 30 on that side is re-tightened to make the corresponding proximal clip 42 away from the distal clip 43 to release the proximal clip 42, and then the control of the proximal clip 42 by the control wire 30 is released again and the valve leaflet is clamped again until the valve leaflet is firmly clamped. When it is determined that the mitral valve repair device 40 clamps the lesion site completely and the mitral regurgitation is significantly improved, the control wire 30 is withdrawn by pulling an end of the control wire 30, making the proximal clip 42 being completely released. Then the delivery device is removed outside of a body to complete the operation. Further, when the control wire 30 is removed from the body, the single control wire 30 is removed more easily. The removal operation is simple. The time consuming is shorter, which is conducive to shortening the operation time.

The components of the mitral valve repair device 40 may be made of biocompatible materials, which may be made of the same or different materials, including but not limited to stainless steel or other metal, cobalt, chromium, nitinol, nickel-titanium, titanium, tantalum and other metal alloys or polymers. In addition, some or all of these components can be made of a biocompatible material that will be absorbed by the surrounding tissue or dissolved into the blood after implantation. The mitral valve repair device 40 was completely surrounded by tissue within several months of implantation, after which the device can be dissolved or be absorbed without negative effects. Specifically, in an embodiment, the proximal clip 42 is a flexible component formed of a shape memory material, specifically nickel-titanium alloy. After the proximal clip 42 formed of the nickel-titanium alloy is heat-set-treated, the proximal clip 42 is expanded outwardly relative to the central base 41 in a normal state, and the proximal clip 42 is tightly attached to the central base 41 through the control wire 30 in the locked state.

Further, in an embodiment, the proximal clip 42 and the distal clip 43 are partially or completely provided with a flow blocking membrane. The flow blocking membrane should be durable to withstand multiple introduction cycles and to withstand the life of cardiac cycles when implanted in a heart. The material of the flow blocking membrane includes, but is not limited to, a single or combination of polyethylene terephthalate, polyester, cotton, polyurethane, expanded polytetrafluoroethylene (ePTFE), silicon, or various biocompatible polymers or fibers. The flow blocking membrane has any suitable form such as fabric, mesh, textured braid, felt, ring structure or porous structure. The flow blocking membrane can also be configured as a friction element to increase adhesion. Drugs, antibiotics, antithrombotic agents, or antiplatelet agents such as heparin or warfarin sodium can be added to the flow blocking membrane. These agents may be immersed in the flow blocking membrane, coated on the flow blocking membrane. The drug can cover the valve leaflets or be absorbed by blood after the valve leaflets are clamped by the mitral valve repair device 40.

In an embodiment, a plurality of connection holes 421 are formed on the proximal clip 421. The control wire 30 sequentially passes through adjacent two connection holes 421 to connect to the proximal clip 421. The number of connection holes 421 is specifically, for example, two to ten. The diameter of the connection hole 421 is from 0.02mm to 3.0mm. In the above embodiments, the control wire 30 is connected and fixed to the proximal clip 42 through the connection holes 421 on the proximal clip 42. In another embodiment, the mitral valve repair device 40 further includes a binding wire. The binding wire ties the control wire 30 to the proximal clip 42. Specifically, the binding wire is made of biocompatible material. When the control wire 30 is fixed on the proximal clip 42 through the binding wire, the proximal clip 42 may not be provided with the connection hole 421. The control wire 30 is directly tied to the proximal clip 42 through the binding wire. Certainly, the proximal clip 42 can also be provided with the connection hole 421. The binding wire passes through the connection hole 421 to tie the control wire 30 to the proximal clip 42. Further, the control wire 30 can be passed through the flow blocking membrane mesh being covered on the proximal clip 42 and fixed with a binding wire to make the connection more secure. Specifically, in the present embodiment, the control wire 30 passes through the first through hole 11 on the delivery sheath 10. The control wire 30 is tied to the proximal clip 42 on the same side by the binding wire of the biocompatible material. Then the control wire 30 passes through the third through hole 21 on the rod 20 to the other side. Similarly, the control wire 30 is tied to the proximal clip 42 on the other side by the binding wire of the biocompatible material, and then returns to the second through hole 12 on the delivery sheath 10.

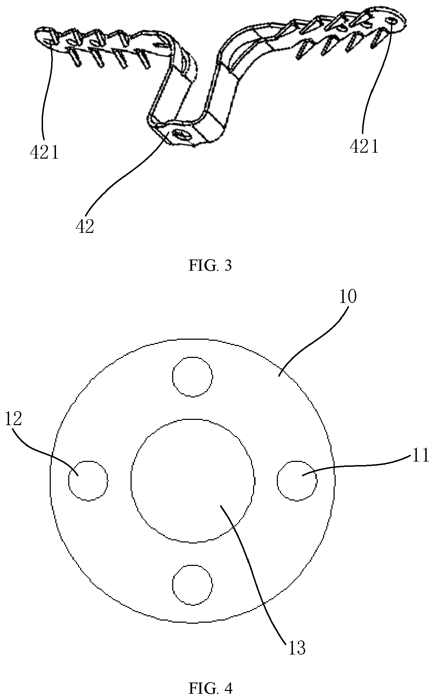

As shown in FIG. 4, the delivery sheath 10 has a main lumen 13 for the rod 20 to extend through. The first through hole 11 and the second through hole 12 are symmetrically arranged relative to the main lumen 13. The cross-sectional shapes of the first through hole 11 and the second through hole 12 can be circular, square, polygonal or other special shapes. The fixing connection between the main lumen 13 and the rod 20 may be, but is not limited to, riveting, bonding, or threaded connection. For convenience of use, the delivery sheath 10 in the present embodiment is provided with two groups of the first through holes 11 and the second through holes 12. In other embodiments, only one group of the first through hole 11 and the second through hole 12 may be provided. More than two groups of the first through holes 11 and the second through holes 12 may also be provided, which is not specifically limited in the present embodiment.

In an embodiment, the rod 20 is provided with a plurality of third through holes 21. The plurality of third through holes 21 are spaced apart along the axial direction of the rod 20. In the present embodiment, the third through holes 21 with different height positions are provided to be adapted to the control wires of a variety of materials and reduce the difficulty of manufacturing the product. Moreover, for the control wires 30 of the same material, the third through holes 21 with different height positions provide more options for passing the control wires 30. The product design is more flexible and the safer.

Specifically, when the control wire 30 of nickel-titanium wire passes through the third through hole 21 on the rod 20 of nickel-titanium and is connected to the proximal clip 42. Since the nickel-titanium wire is characterized as a super elastic material, the nickel-titanium wire will be bent to form a certain curvature in the rod 20, so that an angle is formed between the control wire 30 and the rod 20. The angle formed between the control wire 30 passing through the third through hole 21 at different positions and the rod 20 is different. An appropriate angle can achieve the manipulation of the proximal clip 42 with less force. When the angle between the control wire 30 and the rod 20 is increased, the control wire 30 not only has a tensioning and fixing effect, but also the metal wire with hardness plays a certain pushing role when the proximal clip 42 is released, which can make the proximal clip 42 and the distal clip 43 clamp on the valve leaflet more tightly and firmly, which can help to improve the effect of the operation and the product. With the cooperation of wires of different materials and the third through hole 21 at different positions, the requirements for locking and releasing of the proximal clip 42 are lower, so that the requirements for the strength and thickness of the proximal clip 42 can be reduced. The proximal clip 42 can be selected to be thinner in size, which can increase the feasibility of manufacturing and design, reduce the difficulty of the process, and is conducive to obtaining a more uniform and stable product. Moreover, compared with the proximal clip 42 with a large thickness, the proximal clip 42 with a small thickness can obviously reduce the damage of the device to the valve leaflet during the operation, which is conducive to improving the safety of the device and the operation. In addition, the position of the control wire 30 on the proximal clip 42 remains unchanged. After the angle between the control wire 30 and the rod 20 increases, when the mitral valve repair device 40 is released, the control wire 30 can be pulled out by applying a less force. The swing of the manipulable valve clamp system during the releasing can be reduced. The force on the delivery sheath 10 can also be reduced. The bending of the delivery sheath 10 can be avoided. The manipulation of the device can be effectively improved. The safety of the operation can be improved.

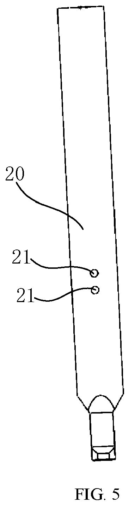

In an embodiment, the angle between the control wire 30 and the rod 20 is from 0° to 150°. In practical application, the number of the third through holes 21 can be selected according to the required angle range. Specifically, the diameter of the third through hole 21 is from 0.02mm to 10mm. The number of the third through holes 21 is from two to twenty. As shown in FIG. 5, in the present embodiment, the number of the third through holes 21 are two, and in other embodiments, the number of the third through holes 21 may be more than two, which is not specifically limited in the present embodiment.

Further, in other embodiments, only one third through hole 21 may be formed in the rod 20 for convenience of processing, which is not specifically limited in the foregoing embodiments.

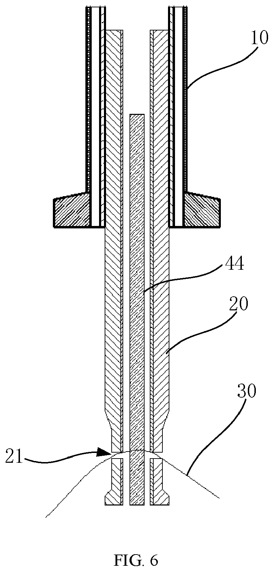

As shown in FIG. 6, in an embodiment, the aperture of the third through hole 21 is slightly larger than the wire diameter of the control wire 30. The control wire 30 located in of the rod 20 is wrapped around a portion of the transmission rod 44. Specifically, the aperture of the third through hole 21 is only slightly larger than the wire diameter of the control wire 30, so that the control wire 30 can just pass through. Certainly, in other embodiments, the aperture of the third through hole 21 may also be equal to the wire diameter of the control wire 30, which is not specifically limited in the present embodiment. In the present embodiment, when the control wire 30 passes through the third through hole 21 and is connected to the proximal clip 42i, the control wire 30 abuts against the inner wall of the rod 20 at the third through hole 21 due to the existence of the curvature. Since the aperture of the third through hole 21 is only slightly larger than the wire diameter of the control wire 30, a frictional force will be generated between the inner wall of the rod 20 at the third through hole 21 and the control wire 30 to lock the control wire 30. The operation of the control wire 30 on one side will not affect the control wire 30 on the other side, and the proximal clip 42 on the other end will not be pulled, so that the single-side valve leaflet can be grasped independently.

Further, the control wire 30 penetrates into the rod 20 from one end of the third through hole 21, passing through the rod 20 from the other end of the third through hole 21 after bypassing the portion of the transmission rod 44. The control wire 30 located in the rod 20 is wrapped around a portion of the transmission rod 44 that is bypassed by the control wire 30, and is located between the transmission rod 44 and the inner wall of the rod 20. A certain frictional force is generated between the transmission rod 44 and the control wire 30. The interference of the transmission rod 44 causes the control wire 30 to be further close to the wall of the rod 20, so that the frictional force between the control wire 30 and the inner wall of the rod 20 can be increased. The control wire 30 is kept locked by the frictional force between the control wire 30 and the transmission rod 44 and the frictional force between the control wire 30 and the inner wall of the rod 20, so that the locking stability of the control wire 30 can be further enhanced, and independent grabbing of one-side leaflet is more reliable. Further, when the mitral valve repair device 40 is released, a pulling force is applied to one end of the control wire 30. The pulling force is greater than the sum of the frictional force between the control wire 30 at the third through hole 21 and the inner wall of the rod 20 and the frictional force between the control wire 30 and the inner walls of the transmission rod 44 and the rod 20, respectively. That is, the control wire 30 can be extracted, and the releasing operation of the mitral valve repair device 40 is completed.

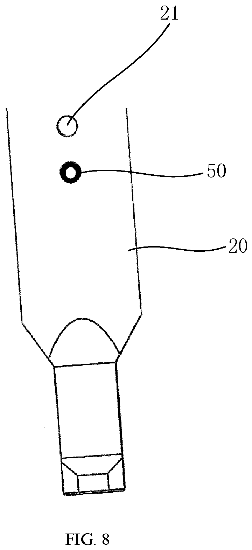

As shown in FIG. 8, in another embodiment, the rod 20 is provided with a second rubber washer 50 at the third through hole 21. The second rubber washer 50 can further reduce the gap between the control wire 30 and the hole wall of the third through hole 21. Moreover, the second rubber washer 50 can increase the frictional force on the control wire 30, so that the control wire 30 does not move without being pulled or pushed, which helps further enhance the locking stability of the control wire 30.

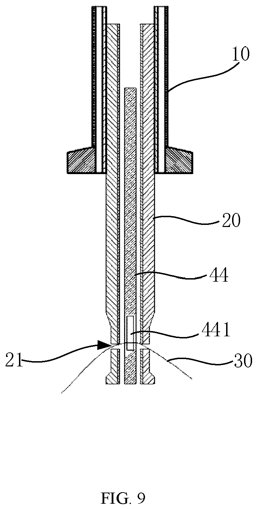

As shown in FIG. 9, in another embodiment, the transmission rod 44 is provided with a keyway 441, and the control wire 30 located in the rod 20 passes through the keyway 441. Specifically, the transmission rod 441 is provided with the keyway 441 for the control wire 30 to pass through. The control wire 30 penetrates into the rod 20 from one end of the third through hole 21, further passes through the keyway 441, then further passes out of the rod 20 from the other end of the third through hole 21. In order to prevent the transmission rod 44 from interfering with the control wire 30, the length of the keyway 441 is greater than the sliding stroke of the transmission rod 44. When the transmission rod 44 is pushed to release the distal clip 43 and the control wire 30 is operated to release the proximal clip 42, a notch of the keyway 441 is aligned with the third through hole 21. The control wire 30 will not have any contact with the rod body of the transmission rod 44, which can avoid the transmission rod 44 from interfering with the control wire 30, and the mitral valve repair device 40 can smoothly release and grasp the valve leaflet. When the single-side valve leaflet is not firmly grasped and needs to be grasped again, the transmission rod 44 is rotated by a preset angle, so that the notch of the keyway 441 and the third through hole 21 are staggered. At this time, the control wire 30 is slightly wrapped around the transmission rod 44 at the keyway 441. Friction forces are generated between the control wire 30 and the transmission rod 44 and between the control wire 441 and the inner wall of the rod 20, respectively, thereby achieving the single-side grasping and locking of the control wire 30, so that the proximal clip 42 can maintain a stable locking state.

Further, as the mitral valve repair device 40 is released, the transmission rod 44 is reversely rotated by a preset angle, so that the notch of the keyway 441 is aligned with the third through hole 21, and then a pulling force is applied to pull out the control wire 30. The withdrawal operation of the control wire 30 is simple and convenient. Further, in the present embodiment, a second rubber washer 50 may be provided on the rod 20 at the third through hole 21 to further enhance the locking stability of the control wire 30. The difference between the manipulatable valve clamping system of the present embodiment and that of the above embodiments is only in that the transmission rod 44 is provided on the keyway 441 to realize the locking of control wire 30. Other structures and compositions are the same, which are not described herein again.

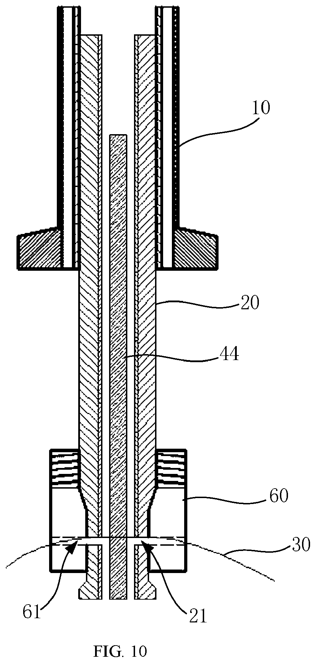

As shown in FIG. 10, in another embodiment, the manipulatable valve clamping system further includes a fixing sleeve 60. The fixing sleeve 60 is sleeved on the outer side of the rod 20. The fixing sleeve 60 is provided with an opening 61 for the control wire 30 to pass through. When the control wire 30 is interlocked with the rod 20, the opening 61 is staggered with the third through hole 21. Specifically, the rod 20 is provided with an external thread, the fixing sleeve 60 is provided with an internal thread, and the fixing sleeve 60 is thread-connected to the rod 20. The fixing sleeve 60 is provided with two openings 61 arranged symmetrically. During assembly of the manipulatable valve clamping system, the two openings 61 are aligned with and in communication with the third through hole 21, so that the control wire 30 can smoothly pass through the rod 20. The control wire 30 is connected to the proximal clip 42 on one side, then passes through the opening 61 on one side, then passes through the third through hole 21, then passes through the opening 61 on the other side and is connected to the proximal clip 42 on the other side. After the assembly of the manipulatable valve clamping system is completed, the fixing sleeve 60 is rotated to stagger the two through holes on the fixing sleeve 60 from the third through hole 21 respectively, so that the control wire 30 respectively abuts against the inner wall of the rod 20 and the inner wall of the fixing sleeve 60, and a friction force is respectively formed between the control wire 30 and the inner wall of the rod 20 and between the control wire 30 and the inner wall of the fixing sleeve 60, so that the control wire 30 is in a locked state. After the mitral valve repair device 40 is placed in the position where the mitral valve cannot be properly closed, the proximal clips 42 at both ends can be released or grasped simultaneously by operating both ends of the control wire 30 simultaneously. When a single-sided valve leaflet needs to be grasped, only operating the control wire 30 at one end will not affect the control wire 30 at the other end, and independent grasping of the single-sided valve leaflet is stable and reliable. Further, when the mitral valve repair device 40 is released, a pulling force is applied to one end of the control wire 30, and the pulling force is greater than the sum of the frictional force between the control wire 30 and the inner wall of the rod 20 and the frictional force between the control wire 30 and the inner wall of the fixing sleeve 60, so that the control wire 30 can be pulled out, and the mitral valve repair device 40 is easily operated to be released.

In an embodiment, a first rubber washer is provided on the fixing sleeve 60 at the opening 61. The first rubber washer can increase the frictional force between the control wire 30 and the inner wall of the fixing sleeve 60, which is conducive to improving the locking stability of the control wire 30. Further, in the present embodiment, the second rubber washer 50 may be provided on the rod 20 at the third through hole 21 to further enhance the locking stability of the control wire 30. The difference between the manipulatable valve clamping system of the present embodiment and that of the above embodiments is only in that the locking of the control wire 30 is realized by providing the fixing sleeve 60. Other structures and compositions are all the same, which are not described herein again.

Each of the technical features of the above-mentioned examples may be combined arbitrarily, to simplify the description, not all the possible combinations of each of the technical features in the above examples are described, however, all of the combinations of these technical features should be considered as within the scope of this application, as long as such combinations do not contradict with each other.

The above embodiments merely illustrate several embodiments of the present application, and the description thereof is specific and detailed, but it shall not be constructed as limiting the scope of the present application. It should be noted that a plurality of variations and modifications may be made by those skilled in the art without departing from the scope of this application, which are all within the scope of protection of this application. Therefore, the protection scope of this application shall be subject to the appended claims.

## Claims

1. A manipulatable valve clamping system, comprising: a delivery sheath (10), a rod (20), a control wire (30), and a mitral valve repair device (40), wherein the mitral valve repair device (40) comprises a central base (41) and proximal clips (42), the rod (20) is connected to the delivery sheath (10), the central base (41) is arranged on the rod (20), the proximal clips (42) are mounted on the central base (41), the delivery sheath (10) is provided with a first through hole (11) and a second through hole (12) arranged symmetrically, the rod (20) is provided with a third through hole (21), the control wire (30) passes through the first through hole (11) and is connected to the proximal clip (42) on a side of the central base (41), the control wire (30) further passes through the third through hole (21) and is connected to the proximal clip (42) on the other side of the central base (41), the control wire (30) further passes out of the delivery sheath (10) through the second through hole (10), and the control wire (30) is configured to be interlocked with the rod (20).

2. The manipulable valve clamping system according to claim 1, wherein the mitral valve repair device (40) further comprises a distal clip (43) and an adjusting mechanism, the adjusting mechanism comprises a transmission rod (44) and an adjusting arm (45), the transmission rod (44) extends through the rod (20) and the central base (41) respectively and is connected to the adjusting arm (45), one end of the distal clip (43) is connected to the central base (41), and the other end of the distal clip (43) is connected to the adjusting arm (45).

3. The manipulable valve clamping system according to claim 2, wherein an aperture of the third through hole (21) is equal to or slightly larger than a wire diameter of the control wire (30), and the control wire (30) located in the rod (20) is wrapped around a portion of the transmission rod (44).

4. The manipulable valve clamping system according to claim 2, wherein transmission rod (44) is provided with a keyway (441), and the control wire (30) located in the rod (20) passes through the keyway (441).

5. The manipulable valve clamping system according to claim 1, further comprising a fixing sleeve (60), wherein the fixing sleeve (60) is sleeved on an outer side of the rod (20), the fixing sleeve (60) is provided with an opening (61) for the control wire (30) to pass through, and when the control wire (30) is interlocked with the rod (20), the opening (61) is staggered with the third through hole (21).

6. The manipulable valve clamping system according to claim 5, wherein a first rubber washer is arranged on the fixing sleeve (60) at the opening (61).

7. The manipulable valve clamping system according to claim 5, wherein the rod (20) is provided with an external thread, the fixing sleeve (60) is provided with an internal thread, and the fixing sleeve (60) is thread-connected with the rod (20).

8. The manipulable valve clamping system according to any one of claims 3 to 7, wherein a second rubber washer (50) is provided on the rod (20) at the third through hole (21).

9. The manipulable valve clamping system according to any one of claims 1 to 7, wherein the proximal clip (42) is provided with a plurality of connection holes (421), and the control wire (30) sequentially passes through adjacent two connection holes (421) to connect to the proximal clip (42).

10. The manipulable valve clamping system according to any one of claims 1 to 7, wherein the mitral valve repair device (40) further comprises a binding wire that ties the control wire (30) to the proximal clip (42).

11. The manipulable valve clamping system according to any one of claims 1 to 7, wherein the rod (20) is provided with a plurality of the third through holes (21), and the plurality of the third through holes (21) are spaced apart along an axial direction of the rod (20).
